# EUROPÄISCHE PATENTSCHRIFT

(11) **EP 3 957 643 B1**
(45) Veröffentlichungstag und Bekanntmachung des Hinweises auf die Patenterteilung: **05.10.2022**
(21) Anmeldenummer: 20191440.5
(22) Anmeldetag: 18.08.2020
(51) Int. Cl.: C07F 9/6574, B01J 31/18, C07C 45/50, C07F 15/00

(54) **NEUE DIPHOSPHITE AUF BASIS VON CIS-BUTENDIOL-1,4**
NEW DIPHOSPHITES BASED ON CIS-BUTENE-1,4-DIOL
NOUVEAUX DIPHOSPHITES À BASE DE CIS-BUTÈNE-1,4-DIOL

(43) Veröffentlichungstag der Anmeldung: 23.02.2022
(73) Patentinhaber: Evonik Operations GmbH, 45128 Essen (DE)
(72) Erfinder: FRANKE, Robert, 45772 Marl (DE); SELENT, Detlef, 18059 Rostock (DE); BÖRNER, Armin, 18059 Rostock (DE); SALE, Anna Chiara, 45657 Recklinghausen (DE)
(74) Vertreter: Evonik Patent Association

(56) Entgegenhaltungen:
- WO-A1-2006/082054
- CN-A- 105 801 625

## Beschreibung

Die Erfindung betrifft neue Diphosphite auf Basis von cis-Butendiol-1,4.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

In CN 105801625A wird ein Herstellungsverfahren für zweizähnige Phosphitliganden und deren Anwendung in der Buchwald-Hartwig-Reaktion beschrieben. Die Liganden weisen bei der Pdkatalysierten Buchwald-Hartwig-Reaktion eine erhöhte Reaktionsaktivität auf.

In WO 2006/082054 A1 werden chirale Diphosphine, Diphosphinite, Diphosphite und Diphosphonite, die von (Z)-2-Butenen abgeleitet sind beschrieben. Diese sind als chirale Liganden geeignet. Die Komplexe zwischen den Diphosphinen, Diphosphiniten, Diphosphiten und Diphosphoniten und Übergangsmetallen können als chirale Katalysatoren in stereokontrollierten Reaktionen verwendet werden.

Die technische Aufgabe der Erfindung ist die Bereitstellung neuer Liganden, welche in der Hydroformylierung von Olefinen eine gesteigerte Selektivität aufweisen.

Die Aufgabe wird gelöst durch eine Verbindung gemäß Anspruch 1.

Verbindung gemäß der Struktur (**I**) oder (**II**): 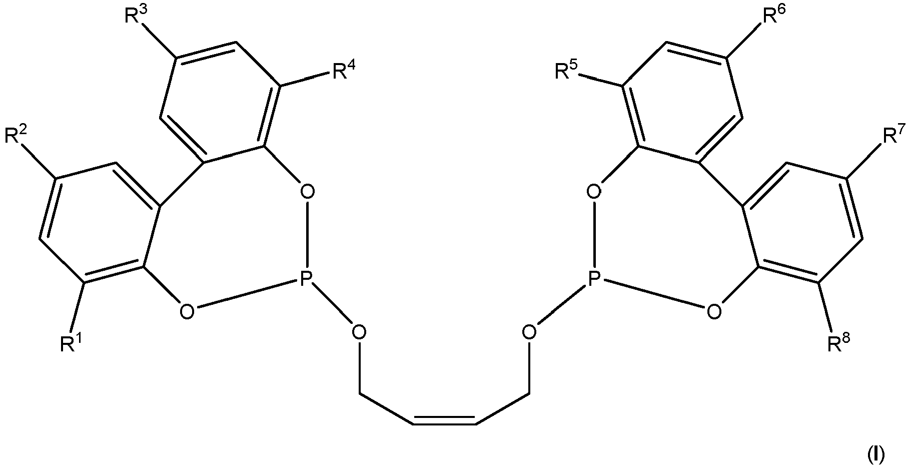 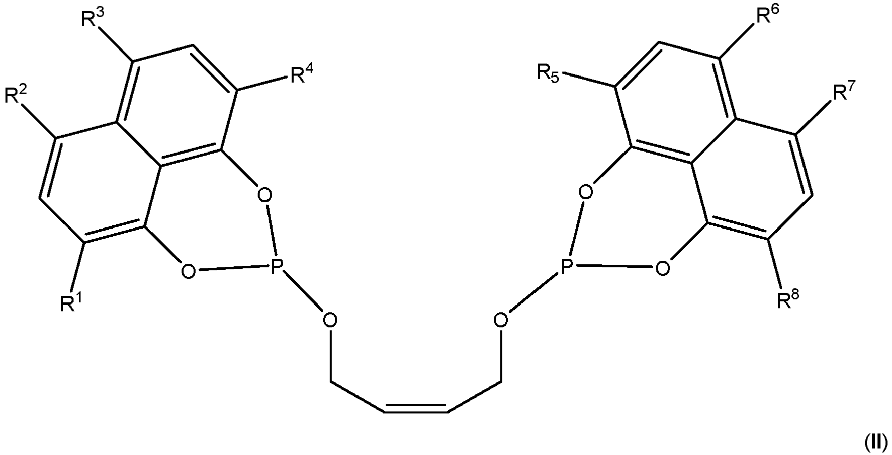 wobei R¹, R², R³, R⁴, R⁵, R⁶, R⁷, R⁸ ausgewählt sind aus: -H, -(C₁-C₁₂)-Alkyl, -O-(C₁-C₁₂)-Alkyl, und die Reste R¹, R², R³, R⁴, R⁵, R⁶, R⁷, R⁸ nicht alle zeitgleich für -*^{t}*Bu stehen.

In einer Ausführungsform sind R¹, R⁴, R⁵, R⁸ ausgewählt aus: -H, -(C₁-C₁₂)-Alkyl.

In einer Ausführungsform stehen die Reste R¹, R⁴, R⁵, R⁸ nicht alle zeitgleich für -*^{t}*Bu.

In einer Ausführungsform stehen die Reste R¹, R⁴, R⁵, R⁸ nicht für -*^{t}*Bu.

In einer Ausführungsform stehet mindestens einer der Reste R¹, R⁴, R⁵, R⁸ für -H.

In einer Ausführungsform stehen R¹, R⁴, R⁵, R⁸ für -H.

In einer Ausführungsform sind R², R³, R⁶, R⁷ ausgewählt aus: -H, -O-(C₁-C₁₂)-Alkyl.

In einer Ausführungsform stehet mindestens einer der Reste R², R³, R⁶, R⁷ für -H.

In einer Ausführungsform stehen R², R³, R⁶, R⁷ für -H.

In einer Ausführungsform weist die Verbindung die Struktur (**I**) auf.

In einer Ausführungsform weist die Verbindung die Struktur (**1**) auf:

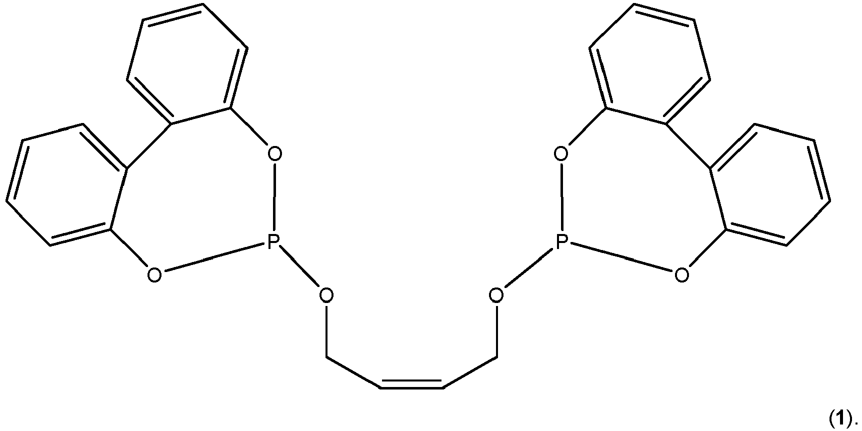

In einer Ausführungsform weist die Verbindung die Struktur (**II**) auf.

In einer Ausführungsform weist die Verbindung die Struktur (**2**) auf:

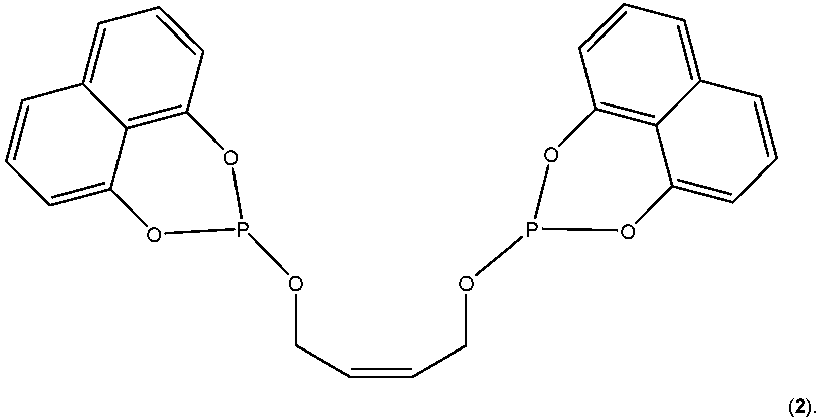

Neben der Verbindung als solche wird auch deren Verwendung zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Des Weiteren wird ein Verfahren beansprucht, in welchem die zuvor beschriebene Verbindung als Ligand eingesetzt wird.

Verfahren umfassend die Verfahrensschritte:
a) Vorlegen eines Olefins,
b) Zugabe einer zuvor beschriebenen Verbindung und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

In einer bevorzugten Ausführungsform ist das Metall Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 60 °C bis 160 °C und ein Druck von 5 bis 70 bar. Besonders bevorzugt sind eine Temperatur von 70 °C bis 140 °C und ein Druck von 15 bis 60 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1- oder 2-Penten, 2-Methyl-1-buten, 2-Methyl-2-buten, 3-Methyl-1-buten, 1-, 2- oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Di-n-buten, Di-iso-buten), Nonene, 2- oder 3-Methyloctene, das bei der Trimerisierung von Propen anfallende C₉-Olefingemisch (Tripropen), Decene, 2-Ethyl-1-octen, Dodecene, das bei derTetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.

Mit dem erfindungsgemäßen Verfahren können unter Verwendung der erfindungsgemäßen Liganden α-Olefine, endständig verzweigte, innenständige und innenständig verzweigte Olefine hydroformyliert werden.

Im Folgenden soll die Erfindung anhand von Ausführungsbeispielen näher erläutert werden.

### Arbeitsvorschriften

### Allgemeine Analytik

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet.

Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR³¹P = SR¹H * (BF³¹P / BF¹H) = SR¹H * 0,4048.

### Synthese (Z)-1,4-bis(dibenzo[d,f][1,3,2]dioxaphosphepin-6-yloxy)but-2-en (1)

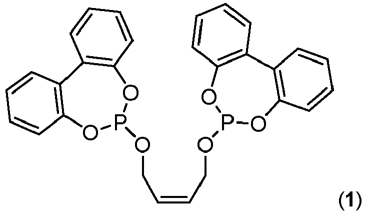

Zu einer auf 0°C gekühlten Mischung von *cis*-1,4-Butendiol (0,206 g; 2,334 mmol) und Triethylamin (1,0 ml) in Toluol (10 ml) wird unter Rühren eine Lösung von 6-Chlorodibenzo[*d*,*f*][1,3,2]dioxaphosphepin (1,253 g; 5,0 mmol) in Toluol (7 ml) getropft. Man lässt auf Raumtemperatur erwärmen, rührt über Nacht, filtriert, und engt das Filtrat bis zur Trockne ein. Der viskose Rückstand wird 4 h bei 50°C/0,1 mbar getrocknet und dann säulenchromatografisch gereinigt (Hexan/Dichlormethan=1:1; Rf=0.5). Ausbeute: 0,733 g (1,42 mmol; 61%). Elementaranalyse (berechnet für C₂₈H₂₂O₆P₂= 516,42 g/mol): C=65,02 (65,12); H=4,22 (4,29); P=11,96 (12,00)%.

ESI-TOF HRMS, *m*/*z*=539,0787; [M+Na⁺]; berechnet *m*/*z*=539,0784.

³¹P-NMR (CD₂Cl₂): 139,4 (s) ppm.

¹H-NMR (CD₂Cl₂): 4,49 (m, 4H); 5,79 (m, 2 H); 7,21-7,56 (m, 16H) ppm.

¹³C-NMR (CD₂Cl₂): 60,1 (d, ²*J*_{CP}=5,7 Hz); 121,8; 125,2; 128,8; 129,4; 130,0; 130,9; 149,8 ppm.

### Synthese (Z)-1,4-bis(naphtho[1,8-de][1,3,2]dioxaphosphinin-2-yloxy)but-2-en (2)

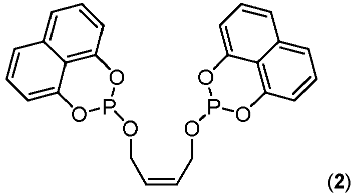

Zu einer auf 0°C gekühlten Mischung von *cis*-1,4-Butendiol (0,230 g; 2,613 mmol) und Triethylamin (1,14 ml) in Toluol (5 ml) wird unter Rühren eine Lösung von 2-Chlornaphtho[1,8-*de*][1,3,2]dioxaphosphinin (1,232 g; 5,487 mmol) in Toluol (16 ml) getropft. Man lässt auf Raumtemperatur erwärmen, rührt über Nacht, filtriert, und engt das Filtrat bis zur Trockne ein. Der Rückstand wird in Acetonitril (9 ml) aufgenommen. Filtration, einengen und trocknen ergibt ein hochviskoses Produkt. Ausbeute: 0,938 g (2,02 mmol; 77%).

Elementaranalyse (berechnet für C₂₄H₁₈O₆P₂= 464,35 g/mol): C=62,16 (62,08); H=3,95 (3,91); P=13,45 (13,34)%.

ESI-TOF HRMS, *m*/*z*=487,0480; [M+Na⁺]; berechnet *m*/*z*=487,0471.

³¹P-NMR (CD₂Cl₂): 112,8 (s) ppm.

¹H-NMR (CD₂Cl₂): 4,34 (m, 4H); 5,52 (m, 2 H); 6,96 (m, 4H); 7,43 (m, 4H); 7,55 (m, 4H) ppm. ¹³C-NMR (CD₂Cl₂): 59,5 (d, ²*J*_{CP}=15,2 Hz); 111,9; 116,5 (d, ²*J*_{CP}=14,3 Hz); 122,1; 127,3; 128,8; 135,0; 144,2 ppm.

### Synthese (Z)-1,4-Bis((4,4,5,5-tetraphenyl-1,3,2-dioxaphospholan-2-yl)oxy)but-2-en (3) (Vergleichsligand)

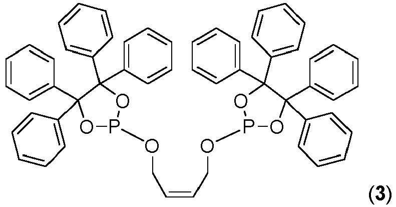

Zu einer auf 0°C gekühlten Mischung von *cis*-1,4-Butendiol (0,091 g; 1,032 mmol) und Triethylamin (0,45 ml) in Toluol (2 ml) wird unter Rühren eine Lösung von 2-Chlor-4,4,5,5-tetraphenyl-1,3,2-dioxaphospholan (0,979 g; 2,271 mmol) in Toluol (5 ml) getropft. Man lässt auf Raumtemperatur erwärmen, rührt über Nacht und filtriert über eine mit Kieselgel beschichtete Fritte und dann zur Trockne eingeengt. Der erhaltene Feststoff wird 4,5 h bei 0,1 mbar/50°C getrocknet und anschließend aus heißem Acetonitril umkristallisiert. Ausbeute: 0,375 g (0,428 mmol; 41%). Elementaranalyse (berechnet für C₅₆H₄₆O₆P₂= 876,92 g/mol): C=76,68 (76,70); H=5,15 (5,29); P=7,03 (7,06)%.

ESI-TOF HRMS, *m*/*z*=899,2689; [M+Na⁺]; berechnet *m*/*z*=899,2667.

³¹P-NMR (CD₂Cl₂): 147,6 (s) ppm.

¹H-NMR (CD₂Cl₂): 4,14 (m, 4H); 5,21 (m, 2 H); 7,07-7,25 (m, 32H); 7,49-7,53 (m, 8H) ppm.

¹³C-NMR (CD₂Cl₂): 59,0 (d, ²*J*_{CP}=24,3 Hz); 94,3 (d, ²*J*_{CP}=8,3 Hz); 127,0 (s, br); 127,2 (s); 128,8 (s, br); 129,7 (s); 142,3 (m); 142,9 (s) ppm.

### Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System gereinigt und unter Argon aufbewahrt. Das als Substrat eingesetzte Olefine 1-Octen (Aldrich) wurde über Natrium am Rückfluss erhitzt und unter Argon destilliert. Im Autoklaven wurden unter Argonatmosphäre Lösungen der Katalysatorvorstufe und des Liganden, jeweils in Toluol, gemischt. Als Katalysatorvorstufe kam [(acac)Rh(COD)] (Umicore, acac=Acetylacetonat-Anion; COD=1,5-Cyclooctadien), zum Einsatz. Der Autoklav wurde bei einem Gesamtgasdruck (Synthesegas: Linde; H₂ (99,999%) : CO (99,997%) = 1:1) von 42 bar für einen Enddruck von 50 bar unter Rühren (1500 U/min) aufgeheizt. Nach Erreichen der Reaktionstemperatur wurde der Synthesegasdruck auf 48,5 bar erhöht und das Olefin mit einem in der Druckpipette eingestellten Überdruck von ca. 3 bar in den Autoklaven gepresst. Die Reaktion wurde bei konstantem Druck, 50 bar (Nachdruckregler der Fa. Bronkhorst, NL), über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 10 ml Pentan verdünnt und gaschromatographisch analysiert: HP 5890 Series II plus, PONA, 50 m × 0,2 mm × 0,5 µm.

### Ergebnisse der Katalyseversuche

[Rh]: 100 ppm, Rh:L = 1:2, p: 50 bar, T: 80 °C; t: 4 h

**Tabelle 1: Hydroformylierung 1-Octen**

| Eintrag | Ligand | Selektivität [%] |
|---|---|---|
| 1 | **1*** | 71,9 |
| 2 | **2*** | 69,7 |
| 3 | **3** | 51,4 |

| | | |
|---|---|---|
| * erfindungsgemäße Verbindung | | |

Mit den erfindungsgemäßen Verbindungen (**1**) und (**2**) konnte eine gegenüber dem Vergleichsliganden (**3**) gesteigerte Selektivität erzielt werden.

Die durchgeführten Versuche belegen, dass die gestellte Aufgabe durch die erfindungsgemäßen Verbindungen gelöst wird.

## Patentansprüche

1. Verbindung gemäß der Struktur (**I**) oder (**II**): 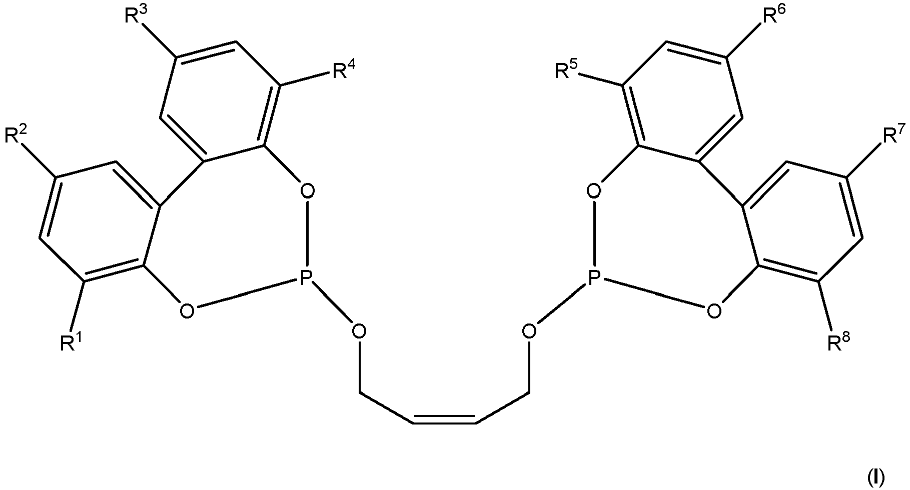 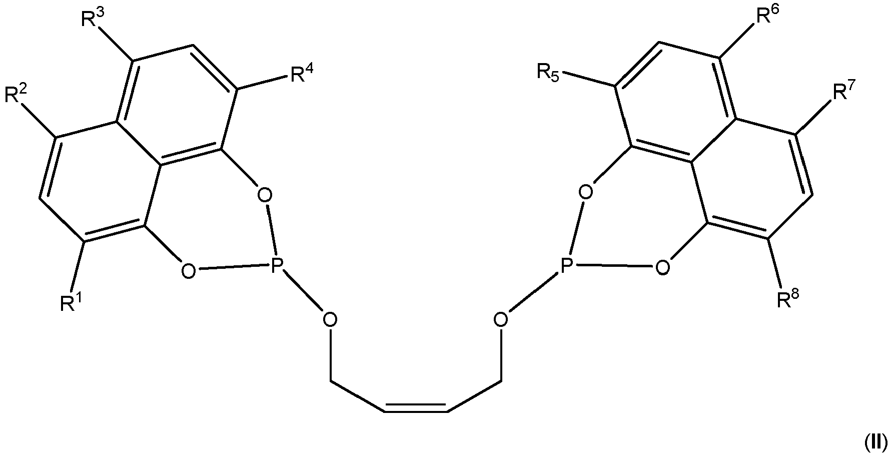 wobei R¹, R², R³, R⁴, R⁵, R⁶, R⁷, R⁸ ausgewählt sind aus: -H, -(C₁-C₁₂)-Alkyl, -O-(C₁-C₁₂)-Alkyl, und die Reste R¹, R², R³, R⁴, R⁵, R⁶, R⁷, R⁸ nicht alle zeitgleich für -*^{t}*Bu stehen.

2. Verbindung nach Anspruch 1,
wobei R¹, R⁴, R⁵, R⁸ ausgewählt sind aus: -H, -(C₁-C₁₂)-Alkyl.

3. Verbindung nach Anspruch 1 oder 2,
wobei die Reste R¹, R⁴, R⁵, R⁸ nicht alle zeitgleich für -*^{t}*Bu stehen.

4. Verbindung nach einem der Ansprüche 1 bis 3,
wobei die Reste R¹, R⁴, R⁵, R⁸ nicht für -*^{t}*Bu stehen.

5. Verbindung nach einem der Ansprüche 1 bis 4,
wobei mindestens einer der Reste R¹, R⁴, R⁵, R⁸ für -H steht.

6. Verbindung nach einem der Ansprüche 1 bis 5,
wobei R², R³, R⁶, R⁷ ausgewählt sind aus: -H, -O-(C₁-C₁₂)-Alkyl.

7. Verbindung nach einem der Ansprüche 1 bis 6,
wobei mindestens einer der Reste R², R³, R⁶, R⁷ für -H steht.

8. Verbindung nach einem der Ansprüche 1 bis 7,
wobei die Verbindung die Struktur (**I**) aufweist.

9. Verbindung nach einem der Ansprüche 1 bis 8,
wobei die Verbindung die Struktur (**1**) aufweist:

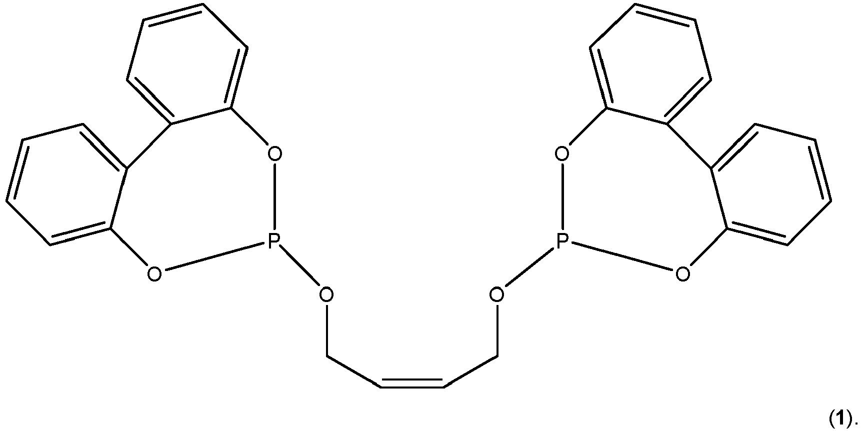

10. Verbindung nach einem der Ansprüche 1 bis 7,
wobei die Verbindung die Struktur (**II**) aufweist.

11. Verbindung nach Anspruch 1 bis 7,
wobei die Verbindung die Struktur (**2**) aufweist:

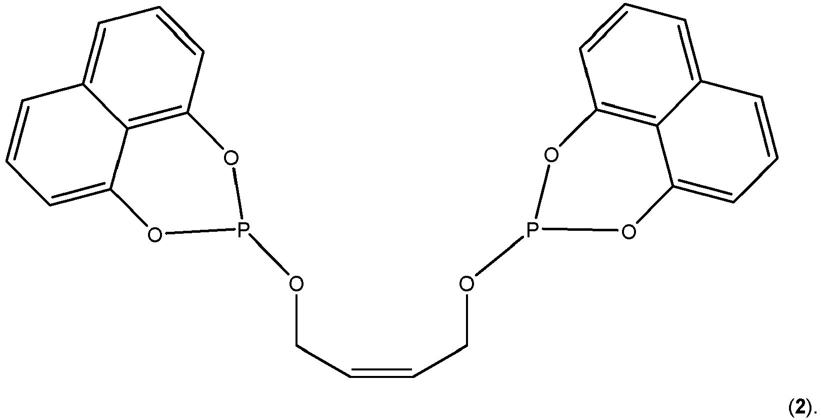

12. Verwendung einer Verbindung nach einem der Ansprüche 1 bis 11,
in einem Ligand-Metall-Komplex zur Katalyse einer Hydroformylierungsreaktion.

13. Verfahren umfassend die Verfahrensschritte:
a) Vorlegen eines Olefins,
b) Zugabe einer Verbindung nach einem der Ansprüche 1 bis 11 und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

## Claims

1. Compound of the structure (**I**) or (**II**): 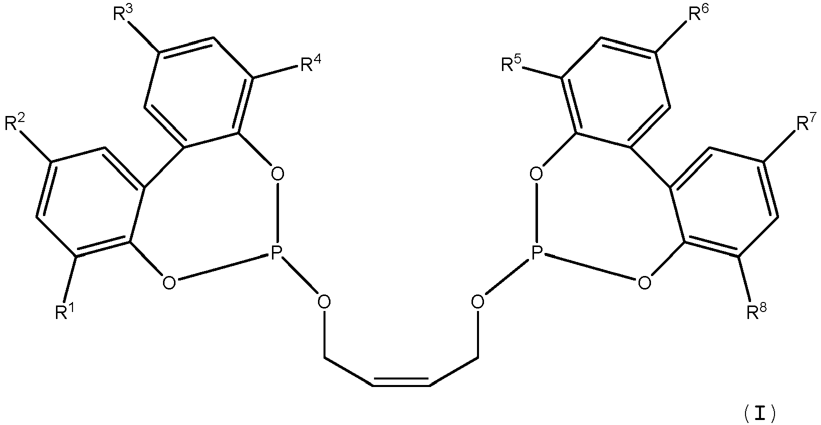 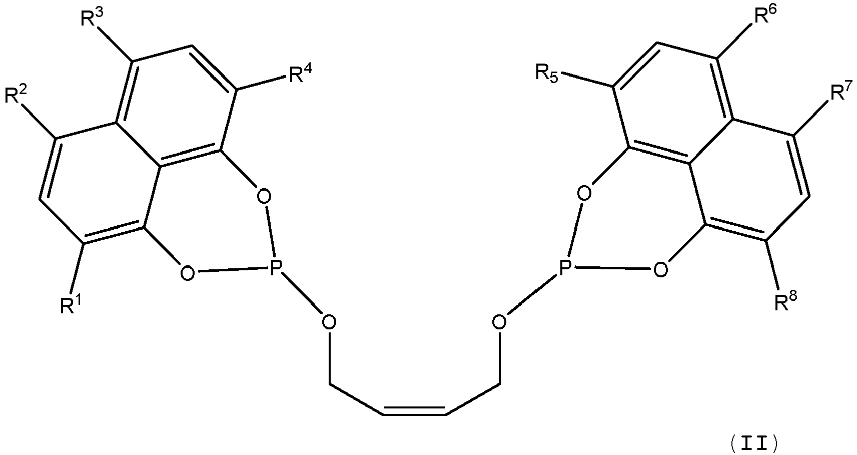 where R¹, R², R³, R⁴, R⁵, R⁶, R⁷, R⁸ are selected from: -H, -(C₁-C₁₂) alkyl, -O-(C₁-C₁₂) alkyl,
and the radicals R¹, R², R³, R⁴, R⁵, R⁶, R⁷, R⁸ are not all simultaneously -*^{t}*Bu.

2. Compound according to Claim 1,
where R¹, R⁴, R⁵, R⁸ are selected from: -H, -(C₁-C₁₂) alkyl.

3. Compound according to Claim 1 or 2,
where the radicals R¹, R⁴, R⁵, R⁸ are not all simultaneously -*^{t}*Bu.

4. Compound according to any of Claims 1 to 3, where the radicals R¹, R⁴, R⁵, R⁸ are not -*^{t}*Bu.

5. Compound according to any of Claims 1 to 4, where at least one of the radicals R¹, R⁴, R⁵, R⁸ is-H.

6. Compound according to any of Claims 1 to 5, where R², R³, R⁶, R⁷ are selected from: -H, -O- (C₁-C₁₂) alkyl.

7. Compound according to any of Claims 1 to 6, where at least one of the radicals R², R³, R⁶, R⁷ is-H.

8. Compound according to any of Claims 1 to 7, where the compound has the structure (I).

9. Compound according to any of Claims 1 to 8, where the compound has the structure (**1**):

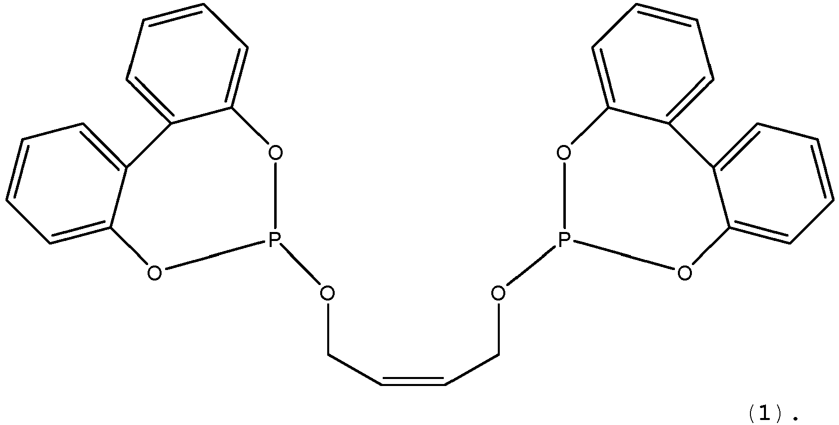

10. Compound according to any of Claims 1 to 7, where the compound has the structure (**II**).

11. Compound according to Claim 1 to 7,
where the compound has the structure (**2**):

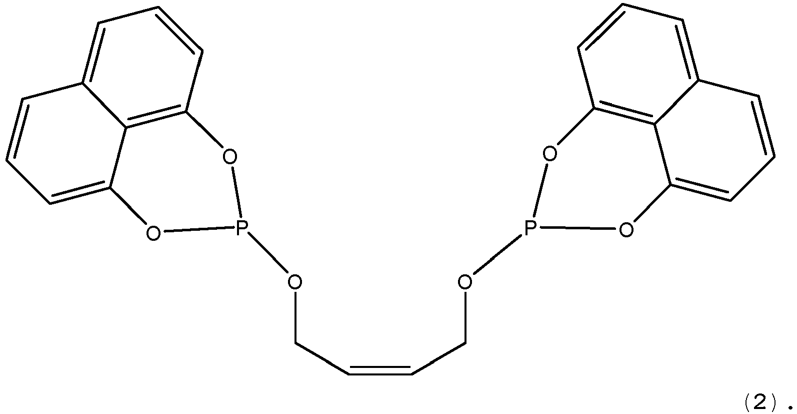

12. Use of a compound according to any of Claims 1 to 11
in a ligand-metal complex for catalysis of a hydroformylation reaction.

13. Process comprising the process steps of:
a) initially charging an olefin,
b) adding a compound according to any of Claims 1 to 11 and a substance comprising a metal selected from: Rh, Ru, Co, Ir,
c) feeding in H₂ and CO,
d) heating the reaction mixture from a) to c), with conversion of the olefin to an aldehyde.

## Revendications

1. Composé selon la structure (I) ou (II) : 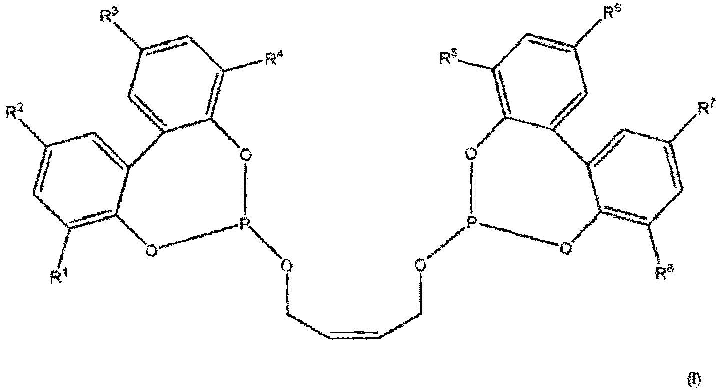 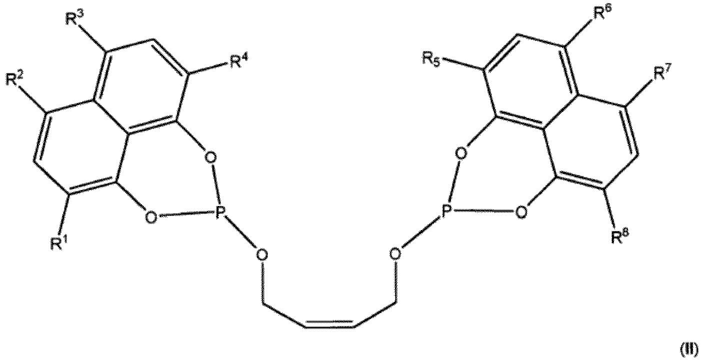 R¹, R², R³, R⁴, R⁵, R⁶, R⁷, R⁸ étant choisis parmi :-H, -(C₁-C₁₂)-alkyle, -O-(C₁-C₁₂)-alkyle, et les radicaux R¹, R², R³, R⁴, R⁵, R⁶, R⁷, R⁸ ne représentant pas tous en même temps -*^{t}*Bu.

2. Composé selon la revendication 1,
R¹, R⁴, R⁵, R⁸ étant choisis parmi : -H, -(C₁-C₁₂)-alkyle.

3. Composé selon la revendication 1 ou 2,
les radicaux R¹, R⁴, R⁵, R⁸ ne représentant pas tous en même temps -*^{t}*Bu.

4. Composé selon l'une quelconque des revendications 1 à 3,
les radicaux R¹, R⁴, R⁵, R⁸ ne représentant pas -*^{t}*Bu.

5. Composé selon l'une quelconque des revendications 1 à 4,
au moins l'un des radicaux R¹, R⁴, R⁵, R⁸ représentant -H.

6. Composé selon l'une quelconque des revendications 1 à 5,
R², R³, R⁶, R⁷ étant choisis parmi : -H, -O-(C₁-C₂)-alkyle.

7. Composé selon l'une quelconque des revendications 1 à 6,
au moins l'un des radicaux R², R³, R⁶, R⁷ représentant -H.

8. Composé selon l'une quelconque des revendications 1 à 7, le composé présentant la structure (I).

9. Composé selon l'une quelconque des revendications 1 à 8, le composé présentant la structure (1) :

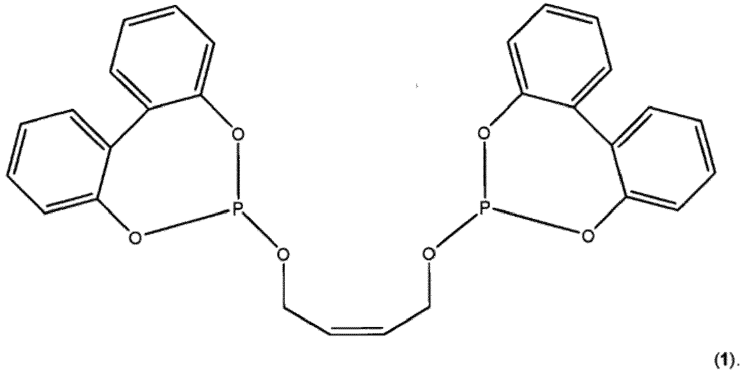

10. Composé selon l'une quelconque des revendications 1 à 7, le composé présentant la structure (II).

11. Composé selon les revendications 1 à 7, le composé présentant la structure (2) :

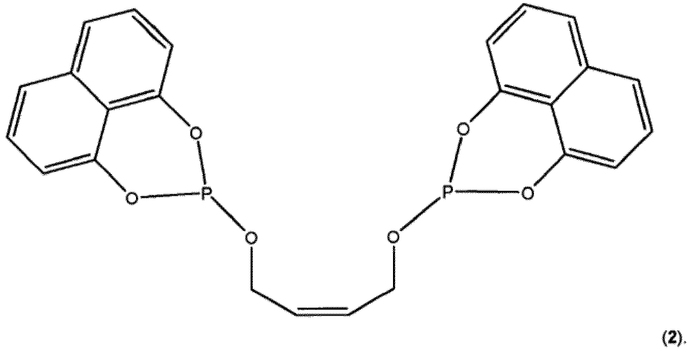

12. Utilisation d'un composé selon l'une quelconque des revendications 1 à 11 dans un complexe de type ligand-métal pour la catalyse d'une réaction d'hydroformylation.

13. Procédé comprenant les étapes de procédé de :
a) chargement d'une oléfine,
b) ajout d'un composé selon l'une quelconque des revendications 1 à 11 et d'une substance qui présente un métal choisi parmi : Rh, Ru, Co, Ir,
c) introduction de H₂ et CO,
d) chauffage du mélange réactionnel de a) à c), l'oléfine étant transformée en un aldéhyde.
